# EUROPÄISCHE PATENTSCHRIFT

(11) **EP 0 360 293 B1**
(45) Veröffentlichungstag und Bekanntmachung des Hinweises auf die Patenterteilung: **30.08.1995**
(21) Anmeldenummer: 89117590.3
(22) Anmeldetag: 22.09.1989
(51) Int. Cl.: C07D 495/04, C07D 211/78, C07D 211/74

(54) **Verfahren zur Herstellung von 4,5,6,7-Tetrahydrothieno-[3,2-c]-Pyridinen**
Process for the preparation of 4,5,6,7-tetrahydrothieno[3,2-c]pyridines
Procédé pour la préparation de 4,5,6,7-tétrahydrothiéno[3,2-c]pyridines

(30) Priorität: 23.09.1988 CH 3539/88
(43) Veröffentlichungstag der Anmeldung: 28.03.1990
(73) Patentinhaber: LONZA AG, CH-3945 Gampel/Wallis (CH)
(72) Erfinder: Gosteli, Jacques, Dr., Basel (CH); Warm, Aleksander, Dr., Visp (Kanton Wallis) (CH)
(74) Vertreter: Weinhold, Peter, Dr.

(56) Entgegenhaltungen:
- EP-A- 0 053 949
- EP-A- 0 170 549
- JOURNAL OF MEDICINAL CHEMISTRY, Band 16, Nr. 6, Juni 1973, Seiten 592-595, Washington, DC, US; M.A. IORIO et al.: "Synthesis, stereochemistry, and analgesic activities of diastereoisomeric esters of 3-allyl-4-phenyl-4-piperidinol"

## Beschreibung

Die Erfindung beinhaltet ein neues Verfahren zur Herstellung von 4,5,6,7-Tetrahydro-thieno-[3,2-c]-pyridinen der Formel 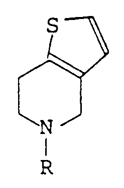
worin R Wasserstoff, eine Formylgruppe, eine Acetylgruppe oder eine Benzoylgruppe ist oder eine Benzylgruppe bedeutet, die gegebenenfalls durch wenigstens ein Halogenatom ringsubstituiert ist. Die Verbindungen der genannten Art haben anti-thrombotische Wirkung, indem sie die Blutplättchenaggregation verhindern (E.Saltiel et al, Drugs, 34 (1987), S.222).

Es sind zahlreiche Verfahren zur Herstellung von Thienopyridinderivaten bekannt. So wird in der DE-PS 25 30 516 eine Synthese beschrieben, in deren Verlauf die Bromierung einer Alkylgruppe in Allyl-Stellung mit N-Bromsuccinimid beschrieben ist. Derartige Reaktionen sind jedoch im technischen Massstab kaum zu realisieren.
Weitere Herstellungswege sind in Bull.Soc.Chim.Belgique 79, (1979) S.301, sowie Arkiv.Kemi.32 (1970) S.217,249, beschrieben. Bei den angegebenen Reaktionsfolgen, die über fünf bzw. sechs Reaktionsschritte verlaufen, werden Acide bzw. komplexe Alkalimetallhydride eingesetzt. Dies ist zwar im Labormassstab realisierbar, lässt sich jedoch nicht auf technische Massstäbe übertragen. Die hohen Kosten der eingesetzten Reagenzien machen zudem beide Verfahren ökonomisch nicht sinnvoll. Die Ausbeuten sind zudem nur sehr gering.
Derivate von 4,5,6,7-Tetrahydro-thieno-[3,2-c]-pyridin, ihre Verwendung in Arzneimitteln und ein Verfahren zu ihrer Herstellung wurden bereits in der DE-PS 24 04 308 beschrieben. Bei diesem Verfahren kondensiert man Verbindungen der Formel 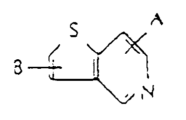
in der A und B jeweils für wenigstens ein Atom bzw. eine Gruppe aus der aus Wasserstoffatomen, Halogenatomen, Hydroxylgruppen, niederen Alkylresten, niederen Alkoxyresten, Nitrogruppen und Aminogruppen bestehenden Gruppe stehen, mit einem Halogenid der Formel Hal-R, in der Hal ein Halogenatom und R ein gegebenenfalls substituierter Alkylrest, Arylrest oder Aralkylrest ist, unter Bildung eines Pyridiniumsalzes der Formel 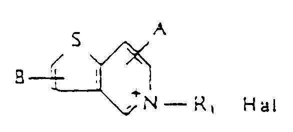
und hydriert anschliessend dieses Pyridiniumsalz zum Derivat der Formel (1).

Dieses Verfahren ist jedoch kostspielig und schwierig durchzuführen, da es zahlreiche und schwierige Verfahrensschritte erfordert.

Aufgabe der Erfindung ist es, diese Nachteile zu vermeiden und ein einfaches Verfahren zur Herstellung der genannten Pyridinderivate zur Verfügung zu stellen.
Ausgangspunkt des erfindungsgemässen Verfahrens sind die 4-Oxo-piperidin-3-carbonsäureester der allgemeinen Formel 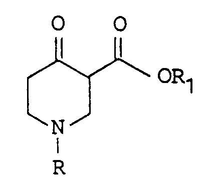
worin R die genannte Bedeutung hat und R₁ für eine Alkylgruppe mit 1 bis 4 C-Atomen steht. Diese Verbindungen sind zugänglich über eine von McElvain et al im J.Am.Chem.Soc. 68, 1946 S.1049 beschriebene Synthese, ausgehend von Ammoniak und Acrylsäureestern.
In der ersten Stufe wird der 4-Oxo-piperidin-3-carbonsäureester mit einem Allylhalogenid in Gegenwart einer Base in die bisher nicht beschriebenen 4-Oxo-3-(2-propenyl)-piperidin-3-carbonsäureester der Formel 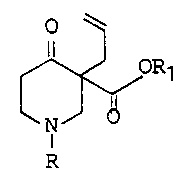
worin R und R₁ die genannte Bedeutung haben, überführt.

Unter Allylhalogenid wird zweckmässig das entsprechende Chlorid, Bromid oder Jodid verstanden.

Als Basen können Alkalihydride, wie z.B. Natrium- oder Kaliumhydrid, Alkalialkoholate wie z.B. Natrium-, Kalium-tert. Butylat, oder auch Alkalicarbonate oder Alkalihydroxide in Verbindung mit einem geeigneten,die Basizität verstärkenden Lösungsmittel, wie z.B. Dimethylformamid, verwendet werden.

Zweckmässig wird in Gegenwart eines Lösungsmittels, das mit den verwendeten Basen inert ist, gearbeitet.

Von Vorteil kann das Arbeiten unter Inertgas, wie Stickstoff oder Argon, sein.

Die Umsetzungstemperaturen bewegen sich zweckmässig zwischen 0°C und dem Siedepunkt des gewählten Lösungsmittels.

Der resultierende 4-Oxo-3-(2-propenyl)-piperidin-3-carbonsäureester kann auf übliche Weise aufgearbeitet und dann der Folgestufe zugeführt werden.

In einer weiteren Möglichkeit kann von einem 3,3′-Iminodipropionsäureester der allgemeinen Formel, 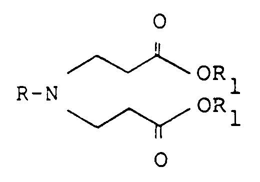
worin R und R₁ die genannten Bedeutungen haben, einer Vorstufe des 4-Oxo-piperidin-3-carbonsäureesters, ausgegangen werden und, ohne Isolierung der letzteren Verbindung, direkt zum 4-Oxo-3-(2-propenyl)-piperidin-3-carbonsäureester gelangt werden.
Dieser Möglichkeit wird der Vorzug gegeben. Besonders bevorzugt wird vom entsprechenden 3,3′-Iminodipropionsäure-tert.-butylester ausgegangen, der direkt in Gegenwart von Kaliumtert.butylat und mit Allylbromid in einem inerten Lösungsmittel zum entsprechenden 4-Oxo-3-(2-propenyl)-piperidin-3-carbonsäure-tert.butylester umgesetzt wird. Ohne dessen Isolierung kann dann die Folgestufe anschliessen.

Der 3,3′-Iminodipropionsäure-tert.butylester seinerseits kann aus Ammoniak durch Umsetzung mit tert.butylacrylat in einer ersten Stufe und Weiterumsetzung mit dem entsprechend Halogenid der allgemeinen Formel

R-Hal

worin R die genannte Bedeutung besitzt und Hal Chlor oder Brom bedeutet, in einer zweiten Stufe hergestellt werden.

Die Folgestufe umfasst die Entfernung der Esterfunktion durch Decarboxylierung und Überführung in die bisher nicht beschriebenen 4-Oxo-3-(2-propenyl)-piperidine der Formel 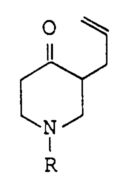
worin R die genannte Bedeutung hat. Die Entfernung der Estergruppe gelingt mit in der Fachwelt an sich bekannten Decarboxylierungsagenzien. Dazu gehören Säuren wie z.B. Salzsäure, Schwefelsäure, Methansulfonsäure oder p-Toluolsulfonsäure, oder Basen wie z.B. Alkalihydroxide oder Alkalialkoholate, oder auch starke Nucleophile wie die Thiolate, die Alkalihalogenide oder -cyanide, gegebenenfalls in Verbindung mit einem die Nucleophilie verstärkenden Lösungsmittel (z.B. Dimethylsulfoxid, Dimethylformamid, Hexamethylphosphorsäuretriamid, N,N′-Dimethylpropylenharnstoff).
Vorzugsweise werden Thiolate in Verbindung mit Hexamethylenphosphorsäuretriamid oder Dimethylpropylenharnstoff angewendet.
Geeignete Thiolate sind vor allem die Alkalialkanthiolate, wie z.B. das Natrium-1-propanthiolat.
Tritt gemäss dem besonders bevorzugten Verfahren als Esterfunktion eine Tert.butylgruppe auf, hat sich als Decarboxylierungsagens verdünnte wässrige Salzsäure als besonders geeignet herausgestellt.

Die Umsetzungstemperatur richtet sich nach dem verwendeten Decarboxylierungsagens. Bei der Verwendung der Thiolate kann zweckmässig zwischen 0 und 40°C, vorzugsweise bei Raumtemperatur, gearbeitet werden. Bei Verwendung von Salzsäure wird zweckmässig bei Rückflusstemperatur gearbeitet.

Die Aufarbeitung des 4-Oxo-3-(2-propenyl)-piperidins kann nach laborüblichen Methoden erfolgen.

Die dritte Stufe des erfindungsgemässen Verfahrens umfasst die Umsetzung des 4-Oxo-3-(2-propenyl)-piperidins in Gegenwart einer Säure mit Ozon und anschliessender Reduktion zum 4-Oxo-3-piperidinacetaldehyd der Formel 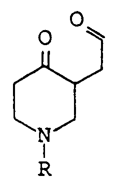
worin R die genannte Bedeutung hat.

Als Säuren sind alle Vertreter, die geeignet sind, mit dem Amin ein Salz zu bilden, denkbar. Besonders vorteilhafte Vertreter sind die Trifluoressigsäure, die Methansulfonsäure oder Chlorwasserstoff.

Um die nach der Ozonisierung entstandenen Ozonide in das gewünschte Aldehyd überzuführen, wird ein in der Fachwelt an sich bekanntes Reduktionsmittel zugesetzt. Geeignete Vertreter sind beispielsweise Triphenylphosphin, Natriumborhydrid, Hydrogensulfite, Thiosulfate oder Dialkylsulfide. Besonders geeignet ist das Dimethylsulfid.

Die Ozonisierung wird vorteilhaft bei Temperaturen zwischen -70 bis +40°C in einer Inertgasatmosphäre und in Gegenwart eines inerten Lösungsmittels durchgeführt.

Das resultierende 4-Oxo-3-piperidinacetaldehyd kann nach laborüblichen Methoden isoliert werden, vorteilhaft wird aber in situ mit Chlorwasserstoff und Schwefelwasserstoff in Gegenwart eines Metallhalogenids der Ringschluss zum Endprodukt vollzogen.

Unter Metallhalogeniden werden zweckmässig die Chloride, Bromide oder Jodide von Zinn, Zink oder Titan verstanden. Vorzugsweise wird Titantetrachlorid, Zinntetrachlorid oder Zinkdibromid eingesetzt.

In der Regel dient als Lösungsmittel dasjenige aus der vorangegangenen Ozonisierung.

Der Ringschluss erfolgt zweckmässig bei Temperaturen zwischen -70 und +40°C, vorzugsweise zwischen -20 und +20°C.

Nach beendeter Reaktion kann das gewünschte 4,5,6,7-Tetrahydro-thieno-[3,2-c]-pyridin auf übliche Weise aufgearbeitet und gereinigt werden.

### Beispiel 1

### a1) Herstellung von Ethyl-1-benzoyl-4-oxo-3-(2-propenyl)-3-piperidincarboxylat

1,16 g (4,22 mmol) Ethyl-1-benzoyl-4-oxo-3-piperidincarboxylat, 9 ml tert. Butanol und 0,57 g (4,93 mmol) Kalium-tert.butylat (97%) wurden bei 25°C vorgelegt und 30 min gerührt. Dann wurden 0,70 g (5,67 mmol) Allylbromid während 2 min zugetropft.
Die Mischung wurde 16 h am Rückfluss gerührt, gekühlt, die Reaktionsmischung eingeengt und zwischen 30 ml Methylenchlorid und 30 ml Wasser verteilt.
Die Emulsion wurde mit Salzsäure (1N) auf pH 7 gestellt. Die Phasen wurden getrennt und die wässrige Phase noch 3 mal mit 30 ml Methylenchlorid extrahiert.
Die vereinigten organischen Phasen wurden mit Magnesiumsulfat getrocknet und eingeengt.
Man erhielt 1,34 g Rohprodukt, welches mittels Säulenchromatographie gereinigt wurde.
Ausbeute: 0,850 g reines Ehtyl-1-benzoyl-4-oxo-3-(2-propenyl)-3-piperidincarboxylat = 64%.
¹H-NMR: (CDCl₃, 300 MHz) δ in ppm
7,45, s, 5H
5,70, bs, 1H
5,07, d, J = 10 Hz, 2H
4,60, bs, 2H
4,18, m, 2H
3,32, m, 2H
2,83, ddd, J = 15 Hz, 11 Hz, 6,5 Hz, 1H
2,52, bs, 3H
1,26, t, J = 7,5 Hz, 3H

### a2) Herstellung von Ethyl-1-[(2-chlorphenyl)methyl]-4-oxo-3-(2-propenyl)-3-piperidincarboxylat

20,00 g (0,067 mol) Ethyl-1-[(2-chlorphenyl)methyl]-4-oxo-3-piperidincarboxylat wurden in 50 ml tert.Butanol vorgelegt und auf 30°C erwärmt. Dann löste man 8,53 g (0,074 mol) Kalium-tert.butylat in 50 ml tert.Butanol auf und erwärmte auf 45°C. Diese Lösung tropfte man bei 30°C während 2 min zum vorgelegten Edukt und rührte noch 1 h bei 30°C. Nun tropfte man 8,50 g (0,67 mol) Allylbromid während 10 min bei 30°C zu und rührte noch während 2 h bei 30°C.
Die Reaktionsmischung wurde am Rotavapor vollständig eingedampft. Der feste Rückstand wurde mit 100 ml Wasser und 100 ml Ether versetzt.
Die Phasen wurden getrennt und die basische Wasserphase noch 2 mal mit 50 ml Ether extrahiert. Dann wurden die vereinigten Etherphasen 1 mal mit 50 ml gesättigter Natriumsulfatlösung gewaschen.
Die organischen Phasen wurden mit Magnesiumsulfat getrocknet und abgedampft. Es resultierte ein gelbes Öl.
Ausbeute: 20,85 g Ethyl-1-[(2-chlorphenyl)methyl]-4-oxo-3-(2-propenyl)-3-piperidincarboxylat = 91,8%.
¹H-NMR: (CDCl₃, 300 MHz) δ in ppm
7,48, d, J = 7,5 Hz, 1H
7,37, d, J = 7,5 Hz, 1H
7,25, q, J = 7,5 Hz, 2H
5,86-5,72, m, 1H
5,03, d, J = 17 Hz, 1H
5,02, d, J = 11 Hz, 1H
4,29-4,07, m, 2H
3,70, s, 2H
3,45, dd, J = 12,5 Hz, 2,5 Hz, 1H
3,10-3,02, m, 1H
2,96-2,84, m, 1H
2,58-2,31, m, 5H
1,23, t, J = 7,5 Hz, 3H

### a3) Herstellung von Methyl-1-[(2-chlorphenyl)methyl]-4-oxo-3-(2-propenyl)-3-piperidincarboxylat

14,60 g (51,8 mmol) Methyl-1-[(2-chlorphenyl)methyl]-4-oxo-3-piperidincarboxylat wurden in 40 ml tert.Butanol gelöst und auf 30°C erwärmt. Innerhalb von 2 min wurden 6,57 (58,4 mmol) Kalium-tert. butylat in 45 ml tert.Butanol zugegeben. Nach 15 min Rühren gab man nochmals 25 ml tert.Butanol zu. Dann wurden 6,34 g (51,4 mmol) Allylbromid innerhalb 10 min zugegeben und während 2 h bei 30-35°C nachgerührt.
Das Reaktionsgemisch wurde eingeengt und zum Rückstand 70 ml Wasser und 70 ml Ether zugegeben.
Die organische Phase wurde mit 50 ml Natriumsulfatlösung gewaschen, über Magnesiumsulfat getrocknet und eingedampft.
Ausbeute: 40,68 g Methyl-1-[(2-chlorphenyl)methyl]-4-oxo-3-(2-propenyl)-3-piperidincarboxylat = 88,1%.
¹H-NMR: (CDCl₃, 300 MHz) δ in ppm
7,47, d, J = 7,5 Hz, 1H
7,37, d, J = 7,5 Hz, 1H
7,25, q, J = 7,5 Hz, 2H
5,85-5,71, m, 1H
5,03, d, J = 17 Hz, 1H
5,02, d, J = 11,5 Hz, 1H
3,71, s, 5H
3,43, dd, J = 11 Hz, 2,5 Hz, 1H
3,09-3,02, m, 1H
2,95-2,84, m, 1H
2,58-2,50, m, 2H
2,47-2,32, m, 3H

### b) Herstellung von 1-[(2-Chlorphenyl)methyl]-3-(2-propenyl)-4-piperidon

0,37 g (1,15 mmol) Methyl-1-[(2-chlorphenyl)methyl]-4-oxo-3-(2-propenyl)-3-piperidincarboxylat, 0,331 g (3,52 mmol) Natrium-1-propanthiolat und 7 ml Hexamethylphosphorsäuretriamid wurden unter Argon vorgelegt und während 2 h bei Raumtemperatur gerührt.
Mit 10 ml Salzsäure 1N wurde die Reaktionslösung angesäuert, dann mit 3 mal 5 ml Ether gewaschen und durch Zugabe von Natriumcarbonatlösung (10% in Wasser) auf pH 9 gestellt.
Darauf wurde mit 35 ml Ether extrahiert, die Etherphase mit 3 mal 5 ml Wasser und 5 ml Natriumsulfatlösung gewaschen, über Magnesiumsulfat getrocknet und eingedampft.
Ausbeute: 0,225 g 1-[(2-Chlorphenyl)methyl]-3-(2-propenyl)-4-piperidon = 74%.
¹H-NMR: (CDCl₃, 300 MHz) δ in ppm
7,52, d, J = 7,5 Hz, 1H
7,38, d, J = 7,5 Hz, 1H
7,25, q, J = 7,5 Hz, 2H
5,80-5,56, m, 1H
5,00, d, J = 19 Hz, 1H
4,98, d, J = 10 Hz, 1H
3,73, 3,72, 2d, AB, J = 14,5 Hz, 2H
3,13-2,98, m, 2H
2,66-2,50, m, 4H
2,45-2,32, m, 2H
2,08, dt, J = 15 Hz, 7,5 Hz, 1H

### c) Herstellung von 1-[(2-Chlorphenyl)methyl]-4-oxo-3-piperidinacetaldehyd

8,1 g (30,7 mmol) 1-[(2-Chlorphenyl)methyl]-3-(2-propenyl)-4-piperidon wurden in 85 ml Methylenchlorid unter Argon vorgelegt und die Lösung auf -60 bis -70°C abgekühlt. Innerhalb von 10 min gab man 7,15 g (61,4 mmol) Trifluoressigsäure in 15 ml Methylenchlorid zu. Darauf leitete man Ozon ein, bis die Lösung eine grünblaue Färbung aufwies.
Das überschüssige Ozon wurde mittels Durchleiten von Stickstoff entfernt.
Danach wurde eine Lösung von 2,36 g (36,8 mmol) Dimethylsulfid in 10 ml Methylenchlorid zugegeben. Nach 5 min Rühren bei -60 bis -70°C wurde auf 0°C erwärmt.
Das Reaktionsgemisch wurde eingeengt und dem Rückstand 300 ml Ether, 150 ml Wasser und 4,2 g Natriumcarbonat zugegeben (pH 8).
Die organische Phase wurde abgetrennt, mit 3 mal 50 ml Wasser und 50 ml Natriumcarbonatlösung gewaschen, über Magnesiumsulfat getrocknet und dann eingedampft.
Ausbeute: 7,07 g 1-[(2-Chlorphenyl)methyl]-4-oxo-3-piperidinacetaldehyd = 76%.
¹H-NMR: (CDCl₃, 300 MHz) δ in ppm
9,29, s, 1H
7,53, d, J = 7,5 Hz, 1H
7,38, d, J = 7,5 Hz, 1H
7,26, q, J = 7,5 Hz, 2H
3,76, s, 2H
3,32-3,15, m, 3H
2,92, ddd, J=17,5 Hz,7,5 Hz,1,5 Hz, 1H
2,72, td, J = 14 Hz, 6 Hz, 1H
2,56, td, J = 11,5 Hz, 4 Hz, 1H
2,38, dt, J = 14 Hz, 2,5 Hz, 1H
2,34-2,22, m, 2H

### d) Herstellung von 5-[(2-Chlorphenyl)methyl]-4,5,6,7-tetrahydro-thieno-[3,2-c]-pyridin

0,97 g (3,68 mmol) 1-[(2-Chlorphenyl)methyl]-3-(2-propenyl)-4-piperidon wurden unter Argon in 10 ml Methylenchlorid gelöst und auf -60 bis -70°C abgekühlt. Innerhalb von 5 min wurden dann 0,86 g (7,39 mmol) Trifluoressigsäure in 2,5 ml Methylenchlorid zugetropft. Anschliessend wurde Ozon eingeleitet, bis die Lösung eine grünblaue Farbe zeigte. Mit Stickstoff wurde das überschüssige Ozon entfernt.
Anschliessend tropfte man innerhalb 5 min eine Lösung von 0,28 g (4,37 mmol) dimethylsulfid in 2,5 ml Methylenchlorid zu. Nach 5 min Rühren bei -60 bis -70°C wurde auf 0°C erwärmt.
Dieser Lösung gab man 5 ml Methanol und danach innerhalb 5 min eine Lösung von 3,39 g (12,88 mmol) Zinntetrachlorid in 5 ml Methylenchlorid zu (exotherm). Bei 0°C begann man mit dem Einleiten von Chlorwasserstoff, 5 min später mit dem Einleiten von Schwefelwasserstoff. Nach 6 h wurde die Einleitung beendet und während 10 h nachgerührt.
Das Reaktionsgemisch wurde eingeengt. Dem Rückstand gab man 75 ml Wasser, 75 ml Ether und Natriumcarbonat zu, bis der pH des Gemisches ca. 9 betrug.
Die organische Phase wurde abgetrennt, mit Magnesiumsulfat getrocknet und eingeengt.
Ausbeute: 0,903 g 5-[(2-Chlorphenyl)methyl]-4,5,6,7-tetrahydro-thieno-[3,2-c]-pyridin = 73%.
¹H-NMR: (CDCl₃, 300 MHz) δ in ppm
7,54, d, J = 7,5 Hz, 1H
7,36, d, J = 7,5 Hz, 1H
7,20, q, J = 7,5 Hz, 2H
7,06, d, J = 5 Hz, 1H
6,70, d, J = 5 Hz, 1H
3,83, s, 2H
3,64, s, 2H
2,88, m, 4H

### e) Herstellung von 5-[(2-Chlorphenyl)methyl]-4,5,6,7-tetrahydro-thieno-[3,2-c]-pyridin

1,35 g (5,13 mmol) Zinntetrachlorid wurden in 10 ml Chloroform vorgelegt. Dazu gab man 0,39 g (1,47 mmol) 1-[(2-Chlorphenyl)methyl]-4-oxo-3-piperidinacetaldehyd, gelöst in 15 ml Chloroform. Nach Abkühlen auf -15°C begann man mit der Einleitung von Chlorwasserstoff und mit der Einleitung von Schwefelwasserstoff. Nach 6 h wurde die Einleitung beendet. Man liess wärend 10 h bei Raumtemperatur nachrühren.
Die Reaktionslösung wurde eingeengt. Dem Rückstand gab man 30 ml Wasser, 30 ml Ether und 1,5 g Natriumcarbonat zu (pH 8-9).
Die organische Phase wurde abgetrennt, über Magnesiumsulfat getrocknet und eingeengt.
Ausbeute: 0,27 g 5-[(2-Chlorphenyl)methyl]-4,5,6,7-tetrahydro-thieno-[3,2-c]-pyridin = 70%.
¹H-NMR: (CDCl₃, 300 MHz) δ in ppm
7,54, d, J = 7,5 Hz, 1H
7,36, d, J = 7,5 Hz, 1H
7,20, q, J = 7,5 Hz, 2H
7,06, d, J = 5 Hz, 1H
6,70, d, J = 5 Hz, 1H
3,83, s, 2H
3,64, s, 2H
2,88, m, 4H

### Beispiel 2

### a) Herstellung von Di-tert.butyl 3,3′-iminodipropionat

112,8 g (0,88 mol) tert.Butylacrylat wurden auf -60°C gekühlt und danach mit 50 g (2,94 mol) Ammoniak versetzt. Das Reaktionsgemisch wurde im Autoklaven während 39 Stunden bei 50°C gerührt. Darauf gab man zusätzlich 32,04 g (0,25 mol) tert.Butylacrylat zu und rührte erneut während 4 Tagen bei 50°C. Das überschüssige tert.Butylacrylat wurde extraktiv zurückgewonnen. Aus dem Reaktionsgemisch wurden durch Extraktion mit Ether 141,26 g eines Gemisches aus Tri-tert.butyl 3,3′,3˝-nitrilotripropionat (NR₃) und Di-tert.butyl 3,3′-iminodipropionat (NR₂) im Verhältnis 1:1,9 erhalten. Dies entsprach einer Ausbeute von 92% bezogen auf tert.Butylacrylat. Dem Gemisch (50 g) wurde zur Trennung 500 ml Ether zugegeben und nach Kühlung auf 0°C 5 g (0,14 mol) HCl-Gas zugegeben. Der resultierende Niederschlag wurde filtiert. Er enthielt 86,3% NR₂ und 13,7% NR₃. Der Niederschlag wurde darauf in 200 ml Wasser gelöst, mit 5n NaOH basisch gestellt und mit 3 x 50 ml Ether extrahiert. Die vereinigten Etherphasen wurden über MgSO₄ getrocknet und eingeengt. Das resultierende Amingemisch wurde erneut mit 4 g HCl behandelt. Der Niederschlag wurde filtriert. Er enthielt nun 99,6% NR₂. Aus dem Niederschlag wurden wie beschrieben 27,23 g des Titelproduktes erhalten.
¹H-NMR: (CDCl₃, 300 MHz)
3,85 (t, J = 7,0 Hz, 4H)
2,43 (t, J = 7,0 Hz, 4H)
1,61 (bs, 1H)
1,47 (s, 18H)

### b) Herstellung von Di-tert.butyl-N-[(2-chlorphenyl)-methyl]-3,3′-iminodipropionat

45,06 g (0,27 mol) 2-Chlorbenzylchlorid und 51 g (0,19 mol) Di-tert.butyl-3,3′-iminodipropionat wurden in 350 ml Acetonitril gelöst, mit 28,3 g (0,28 mol) Triethylamin versetzt und zum Rückfluss erhitzt. Nach 5 Stunden wurden nochmals 8,85 g (0,06 mol) 2-Chlorbenzylchlorid und 9,34 g (0,09 mol) Triethylamin zugegeben. Nach 15 Stunden halten am Rückfluss wurde das Reaktionsgemisch eingeengt. Der Rückstand wurden in 300 ml Wasser, 100 ml HCl 5n und 300 ml Ether aufgenommen. Die saure Wasserphase wurde mit NaOH 20% basisch gestellt und mit 3 x 100 ml Ether extrahiert. Aus der Etherphase wurden 67,7 g (91%) des Titelproduktes erhalten.
¹H-NMR: (CDCl₃, 300 MHz)
7,5 (dd, J = 8,0 Hz, 1,5 Hz, 1H)
7,32 (dd, J = 8,0 Hz, 1,5 Hz, 1H)
7,26-7,14 (m, 2H)
3,7 (s, 2 H)
2,82 (t, J = 7,5 Hz, 4H)
2,41 (t, J = 7,5 Hz, 4H)
1,44 (s, 18H)

### c) Herstellung von 1-[(2-Chlorphenyl)methyl]-3-(-2-propenyl)-4-piperidon

16,60 g (0,145 mol) K-tert.butylat wurden vorgelegt und auf 75 bis 80°C erwärmt. Darauf gab man 50 g (0,125 mol) Di-tert.butyl-N-[(2-chlorphenyl)-methyl]-3,3′-iminodipropionat in 100 ml Toluol innerhalb 10 Minuten zu. Nach 20 Minuten dampfte man das Toluol bei 75°C und 220 mbar praktisch vollständig ab. Dann wurden 400 ml tert.Butanol bei 40°C zugegeben. Zur entstandenen Suspension gab man bei 40°C während 10 Minuten 17,94 g (0,145 mol) Allylbromid zu. Man liess noch 45 Minuten bei 40°C rühren.
Das Reaktionsgemisch wurde nun vollständig eingeengt und dann der feste Rückstand in je 500 ml H₂O + 300 ml Ether gelöst. Die wässrige Phase wurde noch je 2 x mit 100 ml Ether extrahiert. Die vereinigten Etherphasen wurden mit 50 ml gesättigter Na₂SO₄ gewaschen und dann eingeengt. Zum resultierenden Öl wurden dann 500 ml 0,5 n HCl gegeben und während 30 bis 45 Minuten am Rückfluss gehalten (CO₂-Entwicklung).
Das Reaktionsgemisch wurde auf Raumtemperatur abgekühlt und noch mit 20 ml HCl konz. versetzt. Es wurde nun mit 3 x 100 ml Ether extrahiert und die Etherphase mit MgSO₄ getrocknet und abgedampft. Die saure Wasserphase wurde mit NaOH basisch gestellt und 3 x mit 200 ml Ether extrahiert. Die Etherphasen wurden vereinigt und mit MgSO₄ getrocknet und abgedampft. Es resultierte ein gelbliches Öl.
Ausbeute: 28,66 g (87%).
¹H-NMR: (CDCl₃, 300 MHz) δ in ppm
7,52, d, J = 7,5 Hz, 1H
7,38, d, J = 7,5 Hz, 1H
7,25, m, 2H,
5,80-5,56, m, 1H
5,00, d, j= 19,0 Hz, 1H
4,98, d, J = 10,0 Hz, 1H
3,73, 3,72, 2d, AB, J = 14,5 Hz, 2H
3,13-2,98, m, 2H
2,66-2,50, m, 4H
2,45-2,32, m, 2H
2,08, dt, J = 15,0 Hz, 7,5 Hz, 1H
MS: (E.I. 70eV): 263 (M⁺, 2,2), 234 (12), 221 (17,2), 146 (25), 125 (100)
IR (Film): 3073, 2950, 2910, 2802, 2764, 1716

### d) Herstellung von 5-[(2-chlorphenyl)methyl]-4,5,6,7-tetrahydro-thieno[3,2-c]pyridin

13,50 g (51,2 mmol) 1-[(2-chlorphenyl)methyl]-3-(2-propenyl)-4-piperidon wurden unter Argon in 220 ml Methylenchlorid vorgelegt und auf -60°C abgekühlt. Nun gab man 11,93 g (102,4 mmol) Trifluoressigsäure in 30 ml Methylenchlorid zu. Anschliessend begann man mit dem Einleiten von Ozon (25 l O₂/h/0,6 A). Sobald die Lösung grünlich wurde (Überschuss Ozon) stoppte man die Zufuhr von Ozon (30 Minuten). Dann leitete man während 15 Minuten N₂ durch die Lösung, um das überschüssige Ozon auszutreiben (Lösung wurde gelblich). Anschliessend tropfte man während 10 Minuten 3,94 g (61,4 mmol) Dimethylsulfid in 30 ml Methylenchlorid zu. Man rührte noch 5 Minuten bei 60°C und kühlte dann mit Eiswasser auf 0°C. Dann gab man 170 ml Methanol und während 20 Minuten eine Lösung von 34,31 g Titantetrachlorid in 56 ml Methylenchlorid. Jetzt wurde HCl-Gas und 5 Minuten später H₂S-Gas eingeleitet (gelber Niederschlag nach einigen Minuten). Nach 3,5 Stunden wurde die Einleitung von HCl und H₂S abgestellt. Der Kolben wurde verschlossen und über Nacht bei Raumtemperatur stehengelassen. Das Reaktionsgemisch wurde vollständig eingeengt. Dem Rückstand wurden 200 ml Ether + 200 ml 1n HCl zugegeben. Aus der organischen Phase wurden 10,3 g (75%) des Titelproduktes als bräunliches Öl gewonnen.
Zur Herstellung des Hydrochlorids wurde das Rohprodukt in 250 ml Ether gelöst und dann bei 0°C mit HCl-Gas behandelt.
Man erhielt 10,25 g (66%) des Titelproduktes als Hydrochlorid.
Fp. 208 bis 211°C.

## Patentansprüche

1. Verfahren zur Herstellung von 4,5,6,7-Tetrahydro-thieno-[3,2-c]-pyridinen der Formel 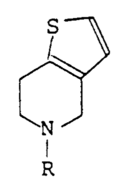 in der R Wasserstoff, eine Formylgruppe, eine Acetylgruppe oder eine Benzoylgruppe ist oder eine Benzylgruppe bedeutet, deren aromatischer Ring gegebenenfalls durch wenigstens ein Halogenatom ringsubstituiert ist, dadurch gekennzeichnet, dass ein 4-Oxo-piperidin-3-carbonsäureester der Formel 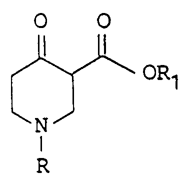 worin R die genannte Bedeutung hat und R₁ für eine Alkylgruppe mit 1 bis 4 C-Atomen steht, mit einem Allylhalogenid in Gegenwart einer Base in einen 4-Oxo-3-(2-propenyl)-piperidin-3-carbonsäureester der Formel 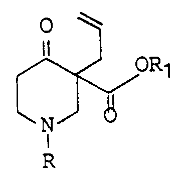 worin R und R₁ die genannte Bedeutung haben, überführt wird, zum 4-Oxo-3-(2-propenyl)-piperidin der Formel 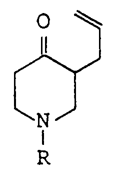 worin R die genannte Bedeutung hat, decarboxyliert wird, in Gegenwart einer Säure mit Ozon umgesetzt und mit einem Reduktionsmittel in das 4-Oxo-3-piperidinacetaldehyd der Formel 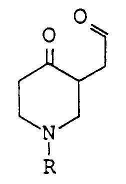 worin R die genannte Bedeutung hat, überführt und schliesslich mit Chlorwasserstoff und Schwefelwasserstoff in Gegenwart eines Metallhalogenids zum Endprodukt ringgeschlossen wird.

2. Verfahren nach Patentanspruch 1, dadurch gekennzeichnet, dass als Base ein Alkalihydrid, ein Alkalialkoholat, ein Alkalihydroxid oder ein Alkalicarbonat verwendet wird.

3. Verfahren nach mindestens einem der Patentansprüche 1-2, dadurch gekennzeichnet, dass die Decarboxylierung mit einer Säure, einer Base oder einem starken Nucleophil erfolgt.

4. Verfahren nach mindestens einem der Patentansprüche 1-3, dadurch gekennzeichnet, dass die Ozonisierung bei Temperaturen zwischen -70 und +40°C durchgeführt wird.

5. Verfahren nach mindestens einem der Patentansprüche 1-4, dadurch gekennzeichnet, dass als Reduktionsmittel Triphenylphosphin, Natriumborhydrid, ein Alkalihydrogensulfit, ein Thiosulfat oder ein Dialkylsulfid, z.B. Dimethylsulfid, eingesetzt wird.

6. Verfahren nach mindestens einem der Patentansprüche 1-5, dadurch gekennzeichnet, dass als Metallhalogenid ein Chlorid, Bromid oder Jodid von Zinn, Zink oder Titan verwendet wird.

7. Verfahren nach mindestens einem der Patentansprüche 1-6, dadurch gekennzeichnet, dass der Ringschluss bei Temperaturen zwischen -70 und +40°C erfolgt.

8. Verfahren nach Patentanspruch 1, dadurch gekennzeichnet, dass der 4-Oxo-3-(2-propenyl)-piperidin-3-carbonsäureester ohne Isolierung der Zwischenstufe ausgehend von einem 3,3′-Iminodipropionsäureester der allgemeinen Formel 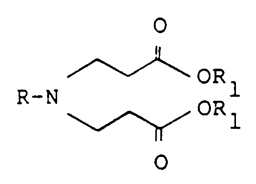 worin R und R₁ die genannten Bedeutungen haben, durch Umsetzung mit einer Base und einem Allylhalogenid gewonnen wird.

9. Verfahren nach Patentanspruch 8, dadurch gekennzeichnet, dass von einem 3,3′-Iminodipropionsäureester der allgemeinen Formel 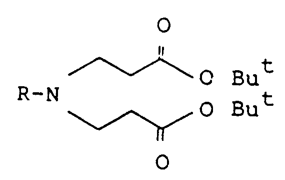 ausgegangen wird, worin R die genannte Bedeutung hat, der durch Umsetzung von Ammoniak mit tert.Butylacrylat und Weiterumsetzung mit einer Verbindung der allgemeinen Formel
R-Hal
worin R die genannte Bedeutung hat und Hal Chlor oder Brom bedeutet, gewonnen wird.

10. 4-Oxo-3-(2-propenyl)-piperidin-3-carbonsäureester der Formel 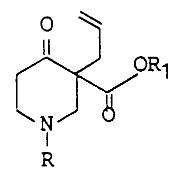 worin R und R₁ die genannte Bedeutung haben.

11. 4-OxO-3-(2-propenyl)-piperidin der Formel 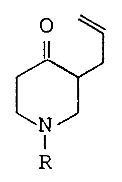 worin R die genannte Bedeutung mit Ausnahme von Benzyl hat.

## Claims

1. Process for the preparation of 4,5,6,7-tetrahydro-thieno-[3,2-c]-pyridines of the formula 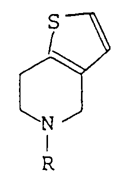 wherein R is hydrogen, a formyl group, an acetyl group, or a benzoyl group or a benzyl group the aromatic ring of which optionally is substituted with at least one halogen atom, characterised in that a 4-oxo-piperidine-3-carboxylic acid ester of the formula 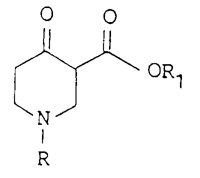 wherein R has the reported meaning and R₁ is an alkyl group having 1 to 4 carbon atoms, is converted with an ally halide in the presence of a base into a 4-oxo-3-(2-propenyl)-piperidine-3-carboxylic acid ester of formula 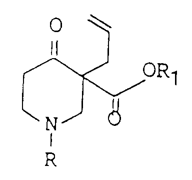 wherein R and R₁ have the reported meanings, is decarboxylated to the 4-oxo-3-(2-propenyl)-piperidine of formula 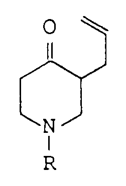 wherein R has the reported meaning, is reacted with ozone in the presence of an acid and with a reducing agent is converted into the 4-oxo-3-piperidine acetaldehyde of formula 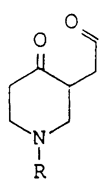 wherein R has the reported meaning, and finally is cyclized with hydrogen chloride and hydrogen sulfide in the presence of a metal halide to give the final product.

2. Process acoording to claim 1, characterised in that an alkali hydride, an alkali alcoholate, an alkali hydroxide, or an alkali carbonate are used as the base.

3. Process according to at least one of claims 1 - 2, characterised in that the decarboxylation is carried out using an acid, a base, or a strong nucleophile.

4. Process according to at least one of claims 1 - 3, characterised in that the ozonization is carried out at a temperature of from -70 to +40 °C.

5. Process according to at least one of claims 1 - 4, characterised in that triphenyl phoshine, sodium borohydride, an alkali hydrogen sulfite, a thiosulfate, or a dialkyl sulfide, e.g. dimethyl sulfide, are used as the reducing agent.

6. Process according to at least one of claims 1 - 5, characterised in that a chloride, bromide, or iodide of tin, zinc or titanium are used as the metal halide.

7. Process according to at least one of claims 1 - 6, characterised in that the cyclization is carried out at temperatures between -70 and +40 °C.

8. Process according to claim 1, characterised in that the 4-oxo-3-(2-propenyl)-piperidine-3-carboxylic acid ester is obtained without the isolation of the intermediate starting from a 3,3'-imino dipropionic acid ester of general formula 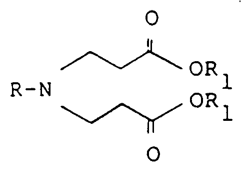 wherein R and R₁ have the reported meanings, through the reaction with a base and an allyl halide.

9. Process according to claim 8, characterised in that one starts with a 3,3'-imino di-propionic acid ester of the general formula 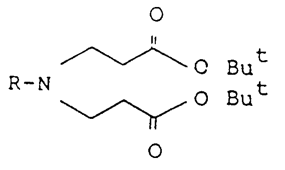 wherein R has the reported meaning, which is obtained by the reaction of ammonia with tert.-butyl acrylate and by further reaction with a compound of general formula
R-Hal
wherein R has the reported meaning and Hal is chlorine or bromine.

10. 4-Oxo-3-(2-propenyl)-piperidine-3-carboxylic acid ester of formula 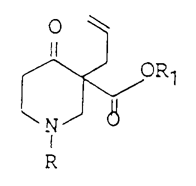 wherein R and R₁ have the reported meaning.

11. 4-Oxo-3-(2-propenyl)-piperidine of the formula 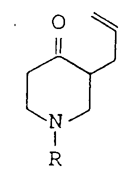 wherein R has the reported meaning with the exception of benzyl.

## Revendications

1. Procédé de préparation de 4,5,6,7-tétrahydrothiéno[3,2-c]pyridines de formule 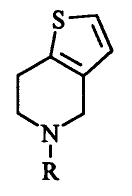 dans laquelle R est un atome d'hydrogène, un groupe formyle, un groupe acétyle ou un groupe benzoyle, ou représente un groupe benzyle dont le noyau aromatique est éventuellement substitué par au moins un atome d'halogène, caractérisé en ce que l'on transforme avec un halogénure d'allyle en présence d'une base un ester d'acide 4-oxopipéridine-3-carboxylique de formule 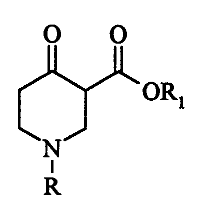 dans laquelle R a la signification indiquée et R₁ représente un groupe alkyle de 1 à 4 atomes de carbone, en un ester d'acide 4-oxo-3-(2-propényl)pipéridine-3-carboxylique de formule 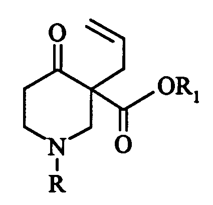 dans laquelle R et R₁ ont la signification indiquée, on le décarboxyle pour donner une 4-oxo-3-(2-propényl)pipéridine de formule 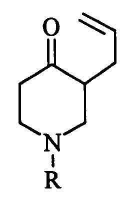 dans laquelle R a la signification indiquée, que l'on fait réagir avec de l'ozone en présence d'un acide et que l'on transforme avec un agent réducteur en le 4-oxo-3-pipéridinacétaldéhyde de formule 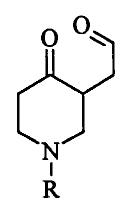 dans laquelle R a la signification indiquée, et enfin on cyclise avec du chlorure d'hydrogène et de l'acide sulfhydrique en présence d'un halogénure métallique pour former le produit final.

2. Procédé selon la revendication 1, caractérisé en ce que l'on utilise comme base un hydrure alcalin, un alcoolate alcalin, un hydroxyde alcalin ou un carbonate alcalin.

3. Procédé selon l'une au moins des revendications 1 - 2, caractérisé en ce que la décarboxylation s'effectue avec un acide, une base ou un nucléophile fort.

4. Procédé selon l'une au moins des revendications 1 - 3, caractérisé en ce que l'ozonisation s'effectue à des températures comprises entre -70°C et +40°C.

5. Procédé selon l'une au moins des revendications 1 - 4, caractérisé en ce que l'on utilise comme agent réducteur la triphénylphosphine, le borohydrure de sodium, un hydrogénosulfite alcalin, un thiosulfate ou un sulfure de dialkyle, par exemple le sulfure de diméthyle.

6. Procédé selon l'une au moins des revendications 1 - 5, caractérisé en ce que l'on utilise comme halogénure métallique un chlorure, un bromure ou un iodure d'étain, de Zinc ou de titane.

7. Procédé selon l'une au moins des revendications 1 - 6, caractérisé en ce que la cyclisation s'effectue à des températures comprises entre -70°C et +40°C.

8. Procédé selon la revendication 1, caractérisé en ce que l'on obtient l'ester d'acide 4-oxo-3-(2-propényl)pipéridine-3-carboxylic, sans isoler le produit intermédiaire, à partir d'un ester d'acide 3,3'-iminodipropionique de formule générale 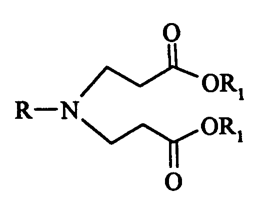 dans laquelle R et R₁ ont les significations indiquées, par réaction avec une base et un halogénure d'allyle.

9. Procédé selon la revendication 8, caractérisé en ce que l'on part d'un ester d'acide 3,3'-iminodipropionique de formule générale 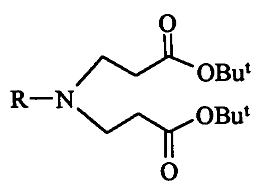 dans laquelle R a la signification indiquée, que l'on obtient par réaction d'ammoniac avec de l'acrylate de t-butyle suivie d'une réaction avec un composé de formule générale
R-Hal
dans laquelle R a la signification indiquée et Hal représente un atome de chlore ou de brome.

10. Ester d'acide 4-oxo-3-(2-propényl)pipéridine-3-carboxylique de formule 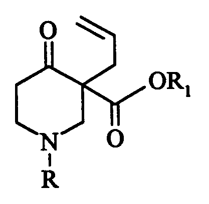 dans laquelle R et R₁ ont la signification indiquée.

11. 4-oxo-3-(2-propényl)pipéridine de formule 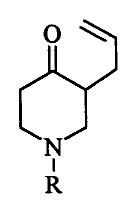 dans laquelle R a la signification indiquée, à l'exception du groupe benzyle.
